# EUROPEAN PATENT SPECIFICATION

(11) **EP 0 786 242 B1**
(45) Date of publication and mention of the grant of the patent: **04.07.2001**
(21) Application number: 96309121.0
(22) Date of filing: 13.12.1996
(51) Int. Cl.: A61F 13/15

(54) **Disposable diaper**
Wegwerfwindel
Couche jetable

(30) Priority: 26.01.1996 JP 1205396
(43) Date of publication of application: 30.07.1997
(73) Proprietor: UNI-CHARM CORPORATION, Kawanoe-shi Ehime-ken (JP)
(72) Inventor: Igaue, Takamitsu, Kawanoe-shi, Ehime-ken (JP); Sasaki, Tohru, Kawanoe-shi, Ehime-ken (JP)
(74) Representative: Parry, Christopher Stephen

(56) References cited:
- GB-A- 2 180 456
- GB-A- 2 286 558

## Description

The present invention relates to a disposable diaper.

It is well known to use a liquid-impermeable plastic film such as polyethylene film as a liquid-impermeable backsheet of a disposable diaper. It is also known from, for example, Japanese Laid-Open Patent Application No. Hei 3-86159 to use a composite sheet composed of a plastic film and a nonwoven fabric bonded to an outer surface of the plastic film as the backsheet.
GB-A-2286558 discloses a coated nonwoven material comprising a base layer made of a fibrous nonwoven web and at least one layer made of a thermoplastic film which has been applied under heat onto one face of the base layer. The coated nonwoven material can be used, in particular, in disposable absorbent hygiene articles such as diapers for babies or incontinent persons as well as training pants and sanitary napkins comprising a liquid impervious backsheet, a liquid permeable topsheet and an absorbent pad disposed between the backsheet and the topsheet and bonded at least to the internal face of the backsheet, for making, in particular, the backsheet which usually is made of a thin polyethylene film to impart to this backsheet a fabric-like or textile appearance and tactile impression, more agreeable for the user.
A diaper of using a plastic film as the backsheet is often disliked by users thereof due to uncomfortable touch peculiar to the plastic film. Use of a composite sheet composed of such a film and a nonwoven fabric covering an outer surface of the film as disclosed in the above-identified applications certainly eliminates the problem peculiar to a plastic film by providing an outer surface of the diaper with a cloth-like touch. However, the backsheet made of such a composite sheet necessarily has a rigidity higher than that of a backsheet made of a plastic film alone and pitches of gathers formed around a waist opening as well as leg openings, respectively, as elastic members provided around those openings contract, become correspondingly rough. When gathers formed around the leg openings are of a rough pitch, in other words, when gathers of a sufficiently fine pitch cannot be obtained around the leg openings, a fitness of the diaper around the legs of a wearer will be deteriorated and, resulting in an undesirable leakage of body fluids around the leg openings.

In view of the problem as has been described above, it is a principal object of the invention to provide a disposable diaper arranged so that a soft touch feeling may be provided around a waist of a wearer and a fitness of the diaper around the legs of a wearer may be improved.

The object set forth above is achieved, according to the invention, by a disposable diaper comprising a liquid-permeable topsheet, a liquid-impermeable backsheet and a liquid-absorbent core disposed between these two sheets, and longitudinally composed of a front body area, a rear body area and a crotch area extending between these two areas, wherein said crotch area is provided along transversely opposite side edge portions with elastically stretchable members destined to surround each leg of a wearer and the backsheet comprises a liquid-impermeable plastic film, the disposable diaper being characterized in that: the backsheet further comprises a pair of nonwoven fabric pieces comprising thermoplastic synthetic fiber integrally bonded to the outer surface of the plastic film over the front and rear body areas respectively, but separated from each other by the crotch area; transversely opposite side edge portions of the crotch area being curved inwardly of said diaper between said pair of nonwoven fabrics.
Embodiments of the invention are described below, with reference to the accompanying drawings, in which:

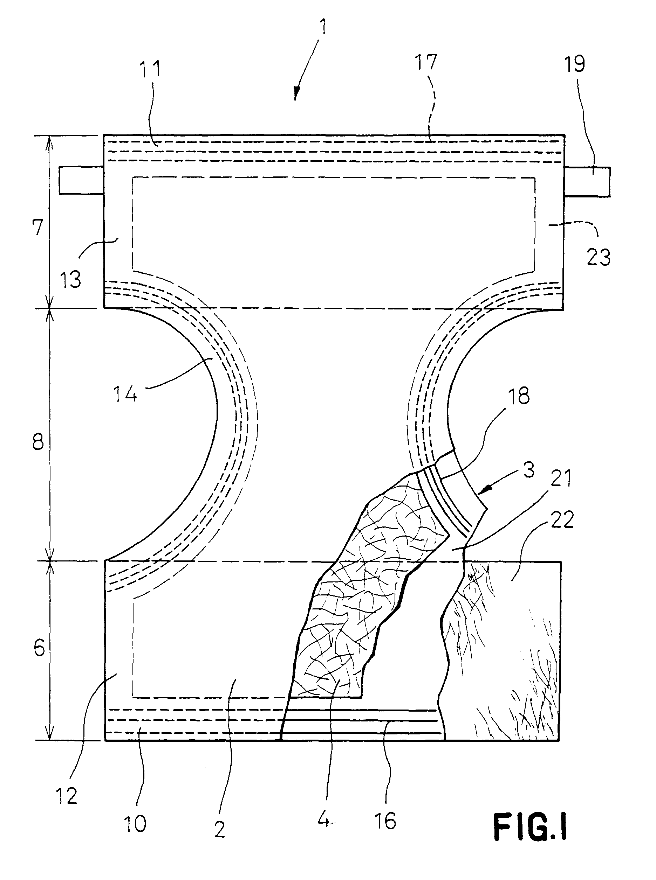

Fig. 1 is a plan view showing a disposable diaper of the invention as partially broken away.
A diaper 1 shown by Fig. 1 in a plan view as partially broken away comprises a liquid-permeable topsheet 2 intended to be contacted with the skin of a wearer, a liquid-impermeable backsheet 3 intended to be non-contacted with the skin and a liquid-absorbent core 4 disposed between these two sheets 2, 3, and is longitudinally composed of a front body area 6, a rear body area 7 and a crotch zone 8 extending between these two areas 6, 7. The topsheet 2 and backsheet 3 are bonded together along their portions extending outward beyond a peripheral edge of the core 4 so as to define longitudinal end portions 10, 11 of the front and rear body areas 6, 7, transversely opposite side edge portions 12,12 and, 13, 13 of the front and rear body areas 6, 7, and transversely opposite side edge portions 14, 14 of the crotch area 8, respectively. Both side edge portions 14, 14 of the crotch area 8 are curved inwardly of the diaper 1 so as to fit around the legs of a wearer. The longitudinal end portions 10, 11 of the front and rear body areas 6, 7 and the side edge portions 14, 14 of the crotch area 8 are provided with elastic members 16, 17 for the waist opening and elastic members 18, 18 for the leg openings, respectively. These elastic members 16, 17 and 18, 18 are disposed between the topsheet 2 and backsheet 3 and bonded to any one of these sheets 2, 3 under elastically stretched conditions. Tape fasteners 19, 19 adapted to be detachably fastened to the side edge portions 12, 12 of the front body area 6 laterally extend from the side edge portions 13, 13 of the rear body area 7, respectively.

The topsheet 2 may be of a liquid-permeable nonwoven fabric or a liquid-permeable perforated plastic film.

The backsheet 3 comprises a liquid-impermeable or air-permeable but liquid-impermeable plastic film 21 covering the entire outer side of the diaper 1, a nonwoven fabric piece 22 substantially identical to the front body area 6 in a shape as well as in a size thereof and integrally bonded to the plastic film 21 over the front body area 6 and a nonwoven fabric piece 23 substantially identical to the rear body area 7 in a shape as well as in a size thereof and integrally bonded to the plastic film 21 over the rear body area 7.

Of the backsheet 3 for the diaper 1, the front and rear body areas 6, 7 adopted to be frequently contacted by hands of a wearer and his or her mother have the outermost surface provided with the nonwoven fabrics 22, 23 which offer soft and agreeable cloth-like touch. The crotch area 8 rarely contacted by the wearer and mother is constituted of the plastic film 21 alone and the absence of the nonwoven fabrics 22, 23 correspondingly reduces rigidity of the backsheet 3 in the crotch area 8 in comparison with the backsheet in the front and rear body areas 6, 7. Consequently, relatively fine gathers can be formed along the transversely opposite side edge portions 14, 14 of the crotch area 8 as the elastic members 18, 18 contract, and such fine gathers ensure a fitness of the diaper 1 around the wearer's legs. With the diaper 1 being in its developed state, the nonwoven fabrics 22, 23 are longitudinally spaced from each other preferably by a range of 100 - 500mm which may be appropriately increased in the case of an adult diaper. Bonding of the nonwoven fabrics 22, 23 to the plastic film 21 may be achieved by an adhesive such as a hot melt type adhesive or heat-sealing just as for bonding of other components of the diaper 1. It is not important whether the nonwoven fabrics 22, 23 should be identical or not to each other in their shapes as well as their sizes.

With the diaper of the invention, the outermost surfaces the front and rear body areas can offer agreeable cloth-like touch, on one hand, and the crotch area can form fine gathers and thereby ensure a fitness around the wearer's legs, on the other hand.

## Claims

1. A disposable diaper (1) comprising a liquid-permeable topsheet (2), a liquid-impermeable backsheet (3) and a liquid-absorbent core (4) disposed between these two sheets, and longitudinally composed of a front body area (6), a rear body area (7) and a crotch area (8) extending between these two areas, wherein said crotch area is provided along transversely opposite side edge portions with elastically stretchable members (18) destined to surround each leg of a wearer and the backsheet (3) comprises a liquid-impermeable plastic film (21), the disposable diaper being characterized in that:
the backsheet (3) further comprises a pair of nonwoven fabric pieces (22,23) comprising thermoplastic synthetic fibers integrally bonded to the outer surface of the plastic film (21) over the front and rear body areas (6, 7) respectively, but separated from each other by the crotch area (8); transversely opposite side edge portions (14, 14) of the crotch area (8) being curved inwardly of said diaper (1) between said pair of nonwoven fabrics (22,23).

2. A disposable diaper as claimed in Claim 1, wherein the pieces (22,23) substantially completely cover the respective body areas and are substantially identical to those areas in shape and size.

## Patentansprüche

1. Wegwerfwindel (1), die eine flüssigkeitsdurchlässige Oberschicht (2), eine flüssigkeitsundurchlässige Unterschicht (3), und einen zwischen diesen beiden Schichten angebrachten flüssigkeitsabsorbierenden Kern (4) umfaßt und in Längsrichtung aus einem Körpervorderseitenbereich (6), einem Körperrückseitenbereich (7) und einem sich zwischen diesen beiden Bereichen erstreckenden Schrittbereich (8) zusammengesetzt ist, wobei der genannte Schrittbereich entlang der einander in Querrichtung gegenüberliegenden Randteile mit elastisch dehnbaren Elementen (18) versehen ist, die dazu bestimmt sind, jeweils ein Bein eines Trägers zu umgeben, und die Unterschicht (3) einen flüssigkeitsundurchlässigen Plastikfilm (21) enthält, wobei die Wegwerfwindel dadurch **gekennzeichnet** ist, daß
die Unterschicht (3) weiterhin ein Paar Vliesstoff-Stücke (22, 23) umfaßt, die mit der äußeren Oberfläche des Plastikfilms (21) über dem Körpervorder- bzw. -rückseitenbereich (6, 7) integral verbundene thermoplastische Synthetikfasern enthalten, die aber voneinander durch den Schrittbereich (8) getrennt sind, wobei die sich in Querrichtung gegenüberliegenden Randteile (14, 14) des Schrittbereichs (8) zwischen dem Paar Vliesstoffe (22, 23) einwärts der Windel (1) gekrümmt sind.

2. Wegwerfwindel nach Anspruch 1, wobei die Stücke (22, 23) die jeweiligen Körperbereiche im wesentlichen vollständig bedecken und mit diesen Bereichen in Form und Größe im wesentlichen identisch sind.

## Revendications

1. Couche jetable (1) comprenant une feuille supérieure perméable au liquide (2), une feuille inférieure imperméable au liquide (3) et un coeur absorbant le liquide (4) disposé entre ces deux feuilles, et composé longitudinalement d'une zone de corps avant (6), une zone de corps arrière (7) et une zone d'entrejambe (8) s'étendant entre ces deux zones, dans laquelle ladite zone d'entrejambe est munie le long des parties de bord latérales transversalement opposées d'éléments élastiquement étirables (18) destinés à entourer chaque jambe d'un porteur, et la feuille inférieure (3) comprend un film plastique imperméable au liquide (21), la couche jetable étant caractérisée en ce que :
la feuille inférieure (3) comprend de plus une paire de pièces de textile non tissé (22, 23) comprenant des fibres synthétiques thermoplastiques liées solidairement à la surface extérieure du film plastique (21) sur les zones avant et arrière (6, 7) du corps respectivement, mais séparées l'une de l'autre par la zone d'entrejambe (8) ; les parties de bord latérales transversalement opposées (14, 14) de la zone d'entrejambe (8) étant incurvées vers l'intérieur de ladite couche (1) entre les pièces de ladite paire de pièces de textile non tissé (22, 23).

2. Couche jetable selon la revendication 1, dans laquelle les pièces (22, 23) couvrent pratiquement complètement les zones respectives du corps, et sont de formes et de dimensions pratiquement identiques à ces zones.
